# EUROPEAN PATENT SPECIFICATION

(11) **EP 2 654 813 B1**
(45) Date of publication and mention of the grant of the patent: **09.03.2016**
(21) Application number: 11824275.9
(22) Date of filing: 21.12.2011
(51) Int. Cl.: A61K 47/32, A61L 26/00

(54) **SPRAYABLE PHARMACEUTICAL COMPOSITIONS FOR TOPICAL APPLICATION COMPRISING SUCRALFATE GEL**
SPRÜHBARE PHARMAZEUTISCHE ZUSAMMENSETZUNGEN ZUR TOPISCHEN ANWENDUNG MIT EINEM SUCRALFATGEL
COMPOSITIONS PHARMACEUTIQUES PULVÉRISABLES POUR APPLICATION TOPIQUE COMPRENANT DU GEL DE SUCRALFATE

(30) Priority: 22.12.2010 IT MI20102366
(43) Date of publication of application: 30.10.2013
(73) Proprietor: Laboratorio Italiano Biochimico Farmaceutico Lisapharma S.P.A., 22036 Erba (IT)
(72) Inventor: COLOMBO, Gaia, I-44100 Ferrara (IT); COLOMBO, Paolo, I-43100 Parma (IT); GALLINA, Laura, I-40138 Bologna (IT); SCAGLIARINI, Alessandra, I-40133 Bologna (IT); SONVICO, Fabio, I-22071 Cadorago (IT)
(74) Representative: Villa, Livia
(86) International application number: PCT/IB2011/055850
(87) International publication number: WO 2012/085859

(56) References cited:
- WO-A1-2010/048981
- US-A- 4 885 281
- US-A1- 2006 024 238

## Description

### FIELD OF THE INVENTION

The present invention relates to pharmaceutical compositions for topical use comprising sucralfate gel, PVA and optionally a vegetable product comprising tocotrienols and tocopherols. In particular, these compositions have proved to be suitable for being sprayed, thus finding advantageous application in the treatment of skin lesions, even very deep lesions, such as second-degree burns.

### STATE OF THE ART

Sucralfate is a sucrose-octasulfate aluminium salt, insoluble in water but soluble in dilute acids and alkali, formed by the salt of sucrose octasulfate and aluminium hydroxide, as shown below:

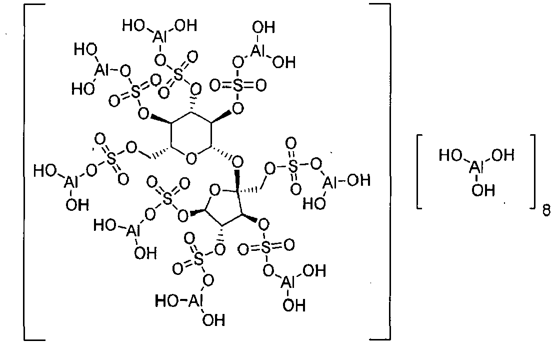

All eight hydroxyl moieties of sucrose are sulfated (sucrose octasulfate, SOS) and each sulfated anion is salinized with the ion (Al(OH)⁵⁺).

Liquid pharmaceutical forms, such as suspensions, are commonly used for therapeutic treatment. The preparation of a stable sucralfate suspension is, however, fairly difficult owing to the tendency of this active ingredient to interact with the commonest suspension agents. The problem of the stabilization of suspensions based on sucralfate has been solved by modifying the physical properties of sucralfate itself; a new physical form of sucralfate has been prepared known as sucralfate gel [Colombo P., Sucralfate gel. The innovative capability of pharmaceutics, Acta Toxicol. Ther. XVI, 1/2, 1995]. The gel is, moreover, characterized by a particle size of a few micrometers, a great affinity for water and distinctive rheological behaviour:
- thixotropic behaviour, an expression of the organization of the particles in flakes and the coagulation of the product which is manifested by a great tendency to adhere to various substrates;
- static flow limit, defined as *spur,* which indicates the rigidity of the system when it is at rest. Once the shear force value corresponding to the *spur* has been exceeded, the structure of the system changes rapidly acquiring greater fluidity. A subsequent rest period restores the previous consistency.
- high dynamic flow limit, an expression of the characteristic three-dimensional structure of gels.

A rheological behaviour of this type is an advantage in the preparation of a stable suspension of sucralfate; in fact, the rigid structure formed during a rest period, makes particle sedimentation impossible, thus maintaining the physical stability of the suspension for a long time. Furthermore, the suspension fluidity, which can be obtained simply by stirring, facilitates the dispensation and uniform distribution of the product.

In 1981, sucralfate was proposed to the scientific community as a mucoprotective agent for the oral treatment of peptic ulcer, based on its cytoprotective properties. Subsequently in 1991, sucralfate was used successfully in peristomal ulcers and in the 2000s, the experience extended to chronic ulcers and burns. In addition to oral formulations, formulations for topical use in the form of creams, ointments, pastes and hydrogels are currently available on the market.

A US patent is known wherein formulations are described in the form of suspensions containing sucralfate, 1-5 wt% of xanthan gum and 1-12.5 wt% of at least one peptizer, on the weight of the sucralfate, where said peptizer can be phosphoric acid, citric acid, other tribasic acids or their salts.

In a recent International Patent Application no. WO 2010/048981, sucralfate gel was used for the topical treatment of skin and mucosal lesions applied by spraying. This application was, however, found to have a number of drawbacks which limited its use. In particular, it was observed that the composition tended to obstruct the spray distributor over time, thus blocking its nozzle. Furthermore, once sprayed the gel showed unsatisfactory adhesion to the skin, tending to form drops. This resulted moreover in a non-uniform, non-homogeneous covering of the lesion to be treated. In addition, once dried, the formation of a crust was observed which, with time and the movements of the person, disadvantageously led to the flaking and detaching of the applied product.

There is therefore a need for a product for topical use which can overcome the drawbacks mentioned above in the products of the prior art.

### SUMMARY OF THE INVENTION

This object was achieved by a composition comprising 15-25 wt% of sucralfate gel, 0.05-0.2 wt% of PVA, 0.5-2 wt% of sodium phosphate monobasic and at least 50 wt% of water, on the total composition weight.

In another aspect, the present invention concerns the use of said composition in the treatment of skin lesions.

As will be clear from the following detailed description, it has been found that the composition of the invention not only supports and promotes re-epithelialization in the case of skin lesions, but also advantageously has an improved spray pattern and superior dermal adhesion, even after drying.

### BRIEF DESCRIPTION OF THE FIGURES

The characteristics and advantages of the present invention will be evident from the following detailed description, from the working examples given for illustrative and non-limiting purposes, and from the annexed Figures, wherein:
- Figure 1 shows the particle size distribution (obtained with a 300 µm lens) of the suspension of sucralfate gel of Example 1;
- Figure 2 shows the degree of sedimentation in relation to the suspension of sucralfate gel of Example 1;
- Figure 3 shows the degree of sedimentation in relation to the suspension of sucralfate gel of Example 4;
- Figure 4 shows the degree of sedimentation in relation to the suspension of sucralfate gel of Example 5;
- Figure 5 shows the average viscosity (Pa*s) of the suspension of sucralfate gel of Example 1;
- Figure 6 shows the average viscosity (Pa*s) of the suspension of sucralfate gel of Example 4;
- Figure 7 shows the average viscosity (Pa*s) of the suspension of sucralfate gel of Example 5;
- Figure 8 shows a comparison product based on sucralfate gel just sprayed on the skin (a) and 20 minutes after spraying (b);
- Figure 9 shows the suspension of sucralfate gel of Example 1 just sprayed on the skin (a) and 10 minutes after spraying (b);
- Figure 10 shows SEM images of a comparison product based on sucralfate gel sprayed directly onto a silicon substrate without further dilution;
- Figure 11 shows SEM images of the suspension of sucralfate gel of Example 1 sprayed directly onto a silicon substrate without further dilution.

### DETAILED DESCRIPTION OF THE INVENTION

The present invention thus relates to a composition comprising 15-25 wt% of sucralfate gel, 0.05-0.2 wt% of PVA, 0.5-2 wt% of sodium phosphate monobasic and at least 50 wt% of water, on the total composition weight.

It was surprisingly found that the selected combination of sucralfate gel and PVA, in the above-mentioned proportions, yields a composition that is easily sprayable, even by manual, non-pressurized sprayers, for which an extremely low risk of nozzle occlusion has been observed, indicating that this composition remains highly stable and optimally fluid over time. Furthermore, as will also be seen from the following Examples, the presence of PVA in the indicated amount has unexpectedly resulted in substantially improving the spray pattern which is extremely homogeneous, and significantly improving the adhesion of the composition to the dermal area to which it is sprayed, even after drying.

The composition preferably comprises 16-22 wt% of sucralfate gel, 0.08-0.15 wt% of PVA, 0.8-1.5 wt% of sodium phosphate monobasic and at least 50 wt% of water, on the total composition weight. More preferably, the composition comprises 17-20 wt% of sucralfate gel, 0.09-0.12 wt% of PVA, 0.9-1.2 wt% of sodium phosphate monobasic and at least 55 wt% of water, on the total composition weight. It has been observed, moreover, that these amounts further improve performance in both the spraying and the dermal adhesion.

According to a preferred embodiment, the composition of the invention comprises 20 wt% of sucralfate gel, 0.1 wt% of PVA, 1 wt% of sodium phosphate monobasic and at least 75 wt% of water, on the total composition weight.

According to another preferred embodiment, the composition of the invention further comprises 0.75-8 wt% of tocotrienols, 0.1-2.5 wt% of tocopherols, and 1-5 wt% of a non-ionic surfactant. It was, in fact, surprisingly found that, in this embodiment, the composition of the invention not only supports and promotes reepithelialization in the case of skin lesions with an improved spray pattern and superior dermal adhesion, but also, due to the presence of tocotrienols and tocopherols, promotes re-epithelialization and increases the elasticity of the skin, particularly desirable in the case of skin lesions such as burns.

Preferably, in this embodiment, the composition comprises 1.5-6 wt% of tocotrienols, 0.4-1.7 wt% of tocopherols, and 2-4 wt% of a non-ionic surfactant. More preferably, the composition comprises 1.5-4.5 wt% of tocotrienols, 0.45-1.4 wt% of tocopherols, and 2.5-3.5 wt% of a non-ionic surfactant.

Within this preferred embodiment, the following are further preferred:
- the composition comprising 18 wt% of sucralfate gel, 1.975 wt% of tocotrienols, 0.575 wt% of tocopherols, 0.1 wt% of PVA, 1 wt% of sodium phosphate monobasic, 3 wt% of a non-ionic surfactant, and at least 72 wt% of water, on the total composition weight; and
- the composition comprising 17 wt% of sucralfate gel, 3.95 wt% of tocotrienols, 1.5 wt% of tocopherols, 0.1 wt% of PVA, 1 wt% of sodium phosphate monobasic, 3 wt% of a non-ionic surfactant, and at least 68 wt% of water, on the total composition weight.

Preferably, said PVA has a number average molecular weight of 9000-80000 Da, more preferably of 10000-50000 Da, and still more preferably of 15000-30000 Da. Optionally, the composition of the invention may also comprise preservatives such as parabens, sorbic acid, and benzoic acid.

Preferably, the composition of the invention consists of 15-25 wt% of sucralfate gel, 0.05-0.2 wt% of PVA, 0.5-2 wt% of sodium phosphate monobasic, 0-9 wt% of tocotrienols, 0-3 wt% of tocopherols, 0-6 wt% of a non-ionic surfactant, and at least 50 wt% of water, the remainder being pharmaceutically acceptable excipients, on the total composition weight. Suitable pharmaceutically acceptable excipients are diluents, disintegrating agents, slip agents, binders, lubricants, stabilizers, adsorbents, release retardants and preservatives.

In another aspect, the present invention relates to the use of the composition described above in the treatment of skin lesions.

For the purposes of the present invention, "skin lesions" are understood to comprise maculae, papulae, vesicles, bullae, pustulae, cysts, erosions, abrasions, rash, ulcers, fissures, sores, telangectasia, scales, erythema, crusts, lichenifications, excoriations, indurations, cuts, lacerations or atrophy.

In particular, said composition is topically sprayed onto the lesion to be treated.

Moreover, as will be seen even more clearly in the following Examples, the composition of the invention allows re-epithelialization of damaged skin, good adherence to skin and excellent tolerability, at the same time a good emollient and moistening capacity.

In another aspect, the invention relates to a process for the preparation of the composition as described above comprising the following steps:
a) providing an aqueous suspension of sucralfate gel and sodium phosphate monobasic;
b) adding to said aqueous suspension, PVA previously dissolved in water at a temperature of 70-100°C under stirring; and
c) homogenizing the mixture resulting from step b) in a high-pressure homogenizer.

In particular, said aqueous suspension of step a) is prepared:
i) by dissolving powdered sucralfate in HCl 2N and adding a solution of NaOH to pH 4.0-4.5 to obtain a gel; and
ii) adding sodium phosphate monobasic under stirring.

According to a preferred embodiment, said process further comprises the following steps:
d) homogenizing in a high-pressure homogenizer tocotrienols and tocopherols dispersed in non-ionic surfactant;
e) adding the mixture of step d) to the aqueous suspension of step a); and
f) homogenizing the mixture resulting from step e) in a high-pressure homogenizer.

The use of a high-pressure homogenizer was found to considerably facilitate particle reduction within the composition, thus leading the formation of a stable homogeneous suspension from a colloidal aqueous suspension.

Preferably, the composition thus obtained undergoes gamma ray sterilization before being transferred to the spray device.

In a further aspect, the present invention relates to a manual pump spray device having a spray actuator and a container, connected to said actuator, comprising the composition of the invention.

Alternatively, the present invention relates to a pressurized spray device having a spray actuator and a container, connected to said actuator, comprising the composition of the invention and at least one propellant agent. A suitable propellant agent is dimethyl ether.

Preferably, the spray device undergoes sterilization, more preferably gamma ray sterilization after being filled with the product.

Working examples of the preparation of compounds according to the present invention are herein below provided for illustrative and non-limiting purposes, as well as examples of evaluation of their efficacy.

### EXAMPLES

### Example 1.

### Preparation of a composition according to the invention

An aqueous suspension was prepared of sucralfate gel, sodium phosphate monobasic and parabens, to which was added PVA previously dissolved in water at a temperature of 70-100°C under stirring.

The mixture thus obtained was placed in a high-pressure homogenizer (PANDA 1 K, GEA Niro Soavi SpA, Parma, IT) to obtain 100 g of final white suspension. Table 1 shows the weight percentages of all the compounds used in the present Example.

**Table 1.**

| | **% w/w** |
|---|---|
| Sucralfate gel (60.2% water) | 20 |
| Propyl p-hydroxy benzoate | 0.02 |
| Methyl p-hydroxy benzoate | 0.2 |
| Anhydrous NaH₂PO₄ | 1 |
| PVA 22000 | 0.1 |
| Purified H₂O | 78.68 |

### Example 2.

### Preparation of a composition according to the invention

The procedure was as per Example 1, but in this case the weight percentages shown in Table 2 were used.

**Table 2.**

| | **% w/w** |
|---|---|
| Sucralfate gel (60.2% water) | 23 |
| Propyl p-hydroxy benzoate | 0.02 |
| Methyl p-hydroxy benzoate | 0.2 |
| Anhydrous NaH₂PO₄ | 1.5 |
| PVA 22000 | 0.15 |
| Purified H₂O | 75.13 |

### Example 3.

### Preparation of a composition according to the invention

The procedure was as per Example 1, but in this case the weight percentages shown in Table 3 were used.

**Table 3.**

| | **% w/w** |
|---|---|
| Sucralfate gel (60.2% water) | 16 |
| Propyl p-hydroxy benzoate | 0.02 |
| Methyl p-hydroxy benzoate | 0.2 |
| Anhydrous NaH₂PO₄ | 0.7 |
| PVA 22000 | 0.07 |
| Purified H₂O | 83.01 |

### Example 4.

### Preparation of a composition according to the invention

Tocotrienols, tocopherols and a non-ionic surfactant were introduced into a high-pressure homogenizer. An aqueous suspension of sucralfate gel, sodium phosphate monobasic and parabens was prepared separately, to which was added PVA previously dissolved in water at a temperature of 70-100°C under stirring.

The homogenized mixture was combined with the aqueous suspension and the whole was again placed in the high-pressure homogenizer to obtain 100 g of final white suspension.

Table 4 shows the weight percentages of all the compounds used in the present Example.

**Table 4.**

| | **% w/w** |
|---|---|
| Sucralfate gel (60.2% water) | 18 |
| Propyl p-hydroxy benzoate | 0.02 |
| Methyl p-hydroxy benzoate | 0.2 |
| Anhydrous NaH₂PO₄ | 1 |
| PVA 22000 | 0.1 |
| tocotrienols | 1.975 |
| tocopherols | 0.575 |
| polysorbate 80 | 3 |
| Purified H₂O | 72.68 |

### Example 5.

### Preparation of a composition according to the invention

The procedure was as per Example 4, but in this case the weight percentages shown in Table 5 were used.

**Table 5.**

| | **% w/w** |
|---|---|
| Sucralfate gel (60.2% water) | 17 |
| Propyl p-hydroxy benzoate | 0.02 |
| Methyl p-hydroxy benzoate | 0.2 |
| Anhydrous NaH₂PO₄ | 1 |
| PVA 22000 | 0.1 |
| tocotrienols | 3.95 |
| tocopherols | 1.15 |
| polysorbate 80 | 3 |
| Purified H₂O | 68.68 |

### Example 6.

### Preparation of a composition according to the invention

The procedure was as per Example 4, but in this case the weight percentages shown in Table 6 were used.

**Table 6.**

| | **% w/w** |
|---|---|
| Sucralfate gel (60.2% water) | 17 |
| Propyl p-hydroxy benzoate | 0.02 |
| Methyl p-hydroxy benzoate | 0.2 |
| Anhydrous NaH₂PO₄ | 1 |
| PVA 22000 | 0.1 |
| tocotrienols | 7.15 |
| tocopherols | 2.1 |
| polysorbate 80 | 3 |
| Purified H₂O | 60.58 |

### Example 7.

### Preparation of a composition according to the invention

The procedure was as per Example 4, but in this case the weight percentages shown in Table 7 were used.

**Table 7.**

| | **% w/w** |
|---|---|
| Sucralfate gel (60.2% water) | 18 |
| Propyl p-hydroxy benzoate | 0.02 |
| Methyl p-hydroxy benzoate | 0.2 |
| Anhydrous NaH₂PO₄ | 1 |
| PVA 22000 | 0.1 |
| tocotrienols | 5.5 |
| tocopherols | 1.6 |
| polysorbate 80 | 3 |
| Purified H₂O | 81.78 |

### Comparative Example 8

A composition of comparison sucralfate gel was prepared, as shown in Table 8.

**Table 8.**

| | **% w/w** |
|---|---|
| Sucralfate gel (60.2% water) | 20 |
| Propyl p-hydroxy benzoate | 0.02 |
| Methyl p-hydroxy benzoate | 0.2 |
| Anhydrous NaH₂PO₄ | 1 |
| Purified H₂O | 78.78 |

### Example 9.

### Characterization of the composition of the invention

The following analyses were conducted to characterize the composition of the invention: particle size distribution, sedimentation stability over time, viscosity, scanning electronic microscope, optical microscope, drying speed, quantity dispensed over time, "spray pattern" and accelerated stability.

### • Particle size analysis by laser light diffraction

Laser diffractometry is a widely used technique in the characterization of materials with dimensions of 0.02-3500 µm. Laser diffraction gives particle size distribution in the form of volume equivalent diameters, i.e. the diameters of spheres equivalent to the particles under examination with the same volume. The results are expressed as cumulative "undersize" distribution.

Using "laser light scattering" (Mastersizer X, Malvern Instruments Ltd., Spring Lane South Malvern, Worcestershire, UK), the particle size distribution in the composition of Example 1 was measured.

The samples were prepared by taking approximately 0.3 g of suspension and dispersing in 10 ml of deionized water (refractive index: 1.33). The samples were stirred manually for a few seconds to disperse the sucralfate gel particles. A cell with a capacity of 100 ml equipped with a sample recirculation and stirring system was used for the analysis. The samples were analysed with 300 µm lenses (range: 1.2 - 600 µm).

Table 1 shows the particle size distributions (expressed as Volume Diameter) of the composition of Example 1.

**Table 1. Particle size distribution of the composition of Example 1**

| Example 1 samples (300 µm lens) | VD 0.1 (µm) | VD 0.5 (µm) | VD 0.9 (µm) |
|---|---|---|---|
| 2 | 1.34 | 3.56 | 7.26 |

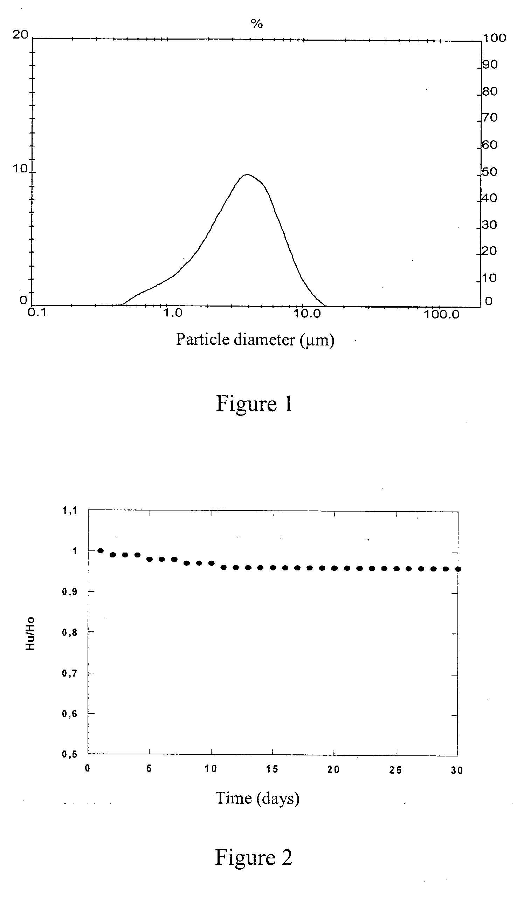

Figure 1 shows the particle size distribution curve of the composition of Example 1, on which eight measurements have been made. The graph shows on the x-axis the volume diameter expressed as µm and on the y-axis the percentage particle population detected.

The data shown in Table 1 and the curve in Figure 1 show an excellent particle size for sucralfate in suspension in the sample of the invention, since, for the spray application, accumulations of particles larger than 300 µm can lead to the occlusion of the dispensing valves of the spray devices.

### • Sedimentation stability over time

Two samples for each of the suspensions of sucralfate of Examples 1, 4 and 5 were placed in graduated cylinders with a capacity of 50 ml and left to stand for 1 month at room temperature. Sediment height,(Hu) and the total suspension height (Ho) were assessed daily to calculate the degree of sedimentation, expressed by the ratio Hu/Ho.

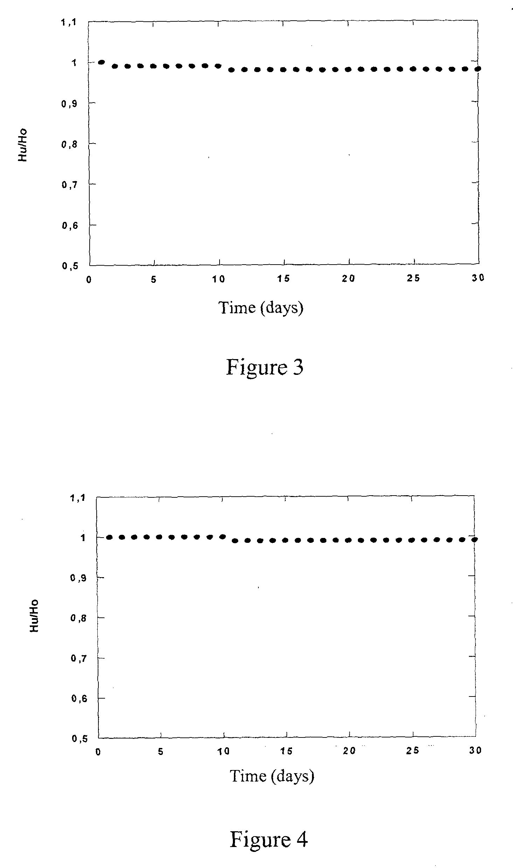

The result of this test is shown in Figures 2, 3 and 4, respectively, which clearly illustrate the high stability over time of the compositions of the invention.

### • Measurement of viscosity

The viscosity tests on samples of Examples 1, 4 and 5 were conducted using a rotating rod viscometer (relative viscometer) VISCOSTAR R (FUNGILAB SPA, Sant Feliu de Llobregat, Barcelona, Spain).

Several viscosity measurements (Pa*s) were made at different rotation values of the auxiliary rotor (from 1 to 200 RPM) to assess the rheological behaviour of the various compositions under examination.

In particular, the samples were mixed for 15 minutes to homogenize the product and they were then left to stand for 15 minutes in a thermostatically-controlled bath. The viscosity was then measured using a rotating viscometer under measuring conditions in which the rotation speed of the measuring device was gradually increased from 1 to 200 RPM and then reduced from 200 to 1 RPM.

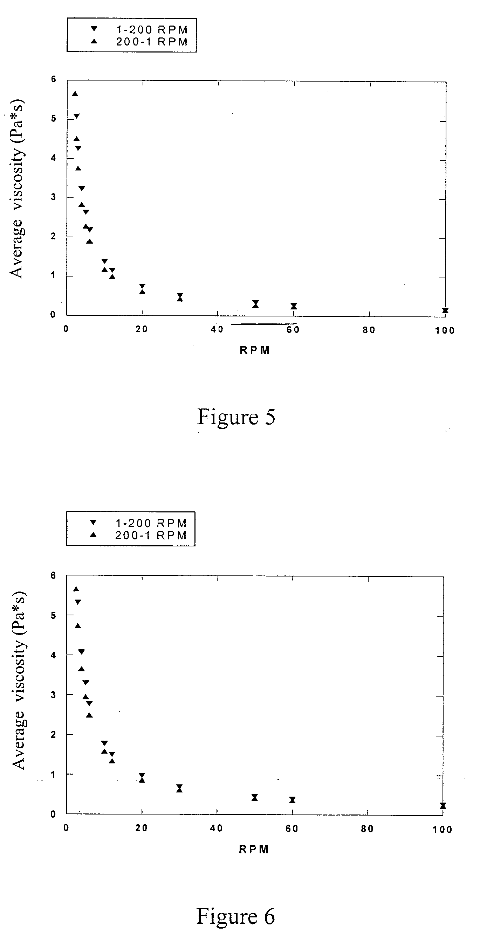

It can be seen from Figures 5-7 that the composition of Example 1 showed lower viscosity than the compositions of Examples 4 and 5.

### • Determination of drying speed

Since a spray product for application for the treatment of skin wounds must attach firmly to the damaged area and must dry quickly to lose the pasty consistency which could easily lead to the removal of the product from the application site, the drying time of the composition of the invention was measured to determine the water loss of the product applied by spraying.

Samples of the composition of Example 1 and samples of sucralfate gel of comparison Example 8 were used.

The samples used in the drying test were prepared by spraying one gram of product, using both manual pump and pressurized devices, onto Petri dishes. The samples thus prepared were placed in an oven at 32±1 °C. The weight loss of the product was recorded every 5 minutes for the first hour and every 15 minutes for the second and third hour.

The result was calculated as percentage loss m/m, i.e. as weight loss % and H₂O loss % as a function of time.

The drying of the spray formulations showed a continual reduction in the water content of the sucralfate gel layer applied by spraying onto the Petri dish. Analysing the two resulting curves, after 50 minutes the composition of Example 1 showed weight loss of 50% (evaporation of 63% of the water), while the comparison sucralfate gel showed weight loss of 35% (evaporation of 45% of the water).

Finally after 100 minutes, the composition of Example 1 showed weight loss of 75% (evaporation of 95% of the water), while the comparison sucralfate gel showed weight loss of 60% (evaporation of 80% of the water).

This demonstrated that the composition of the invention had greater drying speed due to considerable loss of water content over time.

Drying tests were also performed on the same compositions as above, spraying them onto the skin of healthy volunteers, using manual pump devices.

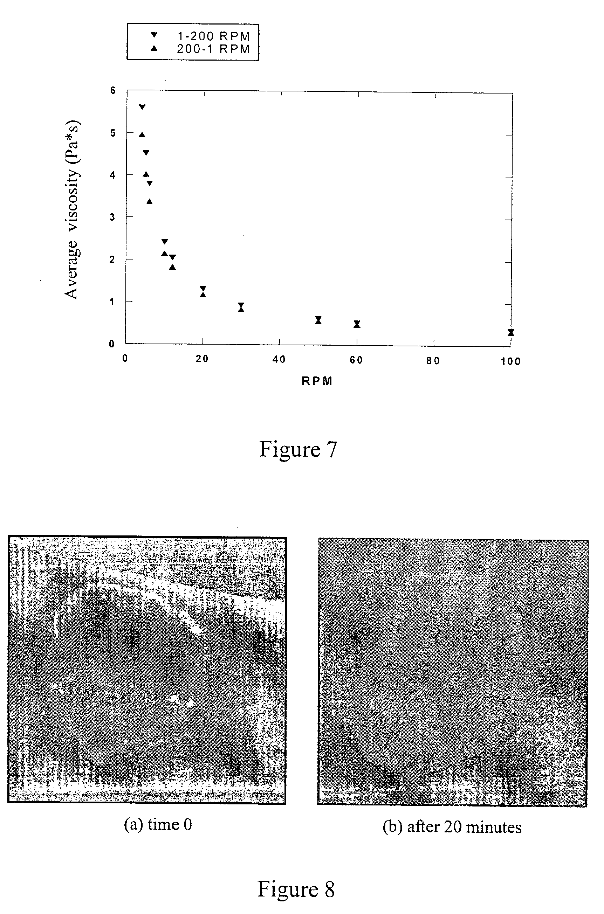

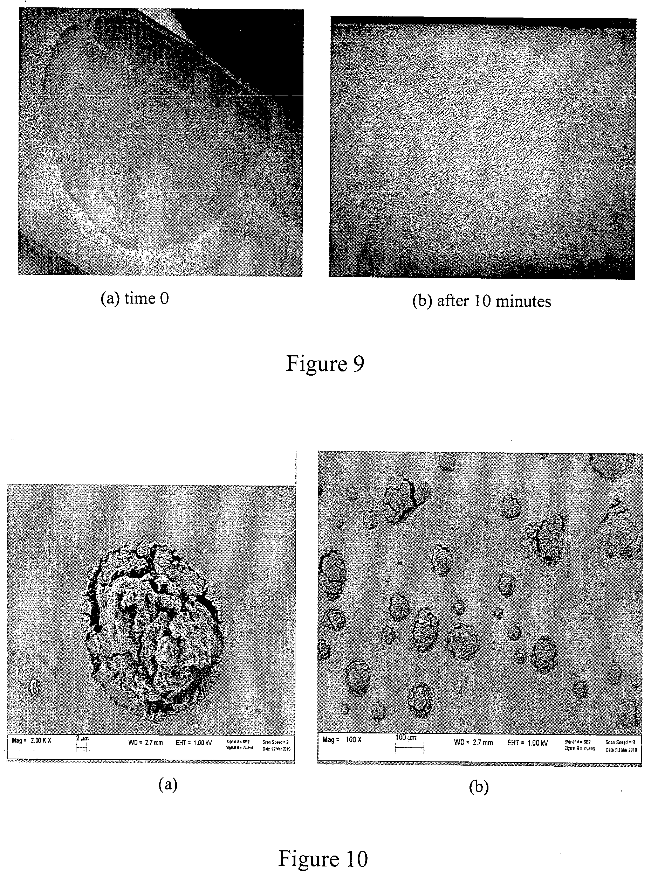

Figures 8 and 9 show the deposits of the compositions after spraying at time zero and after a period of drying.

It was observed that the comparative sucralfate gel, in contact with the skin (time 0), did not adhere well to the skin and tended to form drops. The composition of the invention, conversely, in contact with the skin generated a dried product that was more covering, strongly adhering and more uniform.

This difference became more apparent by comparing the two products dispensed after they had dried on the skin; moreover in Figure 8b, the drying of the comparative sucralfate gel formed a crust which, with time and the movements of the person, disadvantageously led to the flaking and detaching of the applied product; conversely in Figure 9b, the drying of the composition of the invention occurred in half the time and formed a more elastic, better adhering crust, which thus adapted advantageously to the movements and twisting of the skin without unwanted detachments occurring.

### • SEM (Scanning Electron Microscope) Analysis

SEM analysis of the compositions of the invention and comparative compositions sprayed directly onto a silicon substrate without further dilution revealed that:
- in Figures 10a and 10b the dried comparative sucralfate gel showed non-homogeneous crusty fragments,

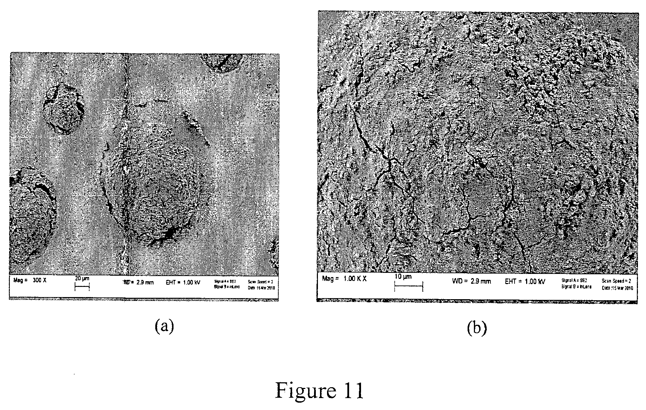

- in Figures 11 a and 11 b the composition of Example 1 dried was clearly more compact and moreover, when sprayed onto the skin, it dried quickly and adhered far better to the skin.

The composition of the invention consequently demonstrated optimal technical characteristics for administration by a spray device for the treatment of skin lesions. Homogenization of the suspension of sucralfate gel with a high-pressure homogenizer led to a significant reduction in the size of the suspended particles and to a more uniform, fluid product being obtained which is therefore easier to dispense from the spray devices, thus significantly reducing the risk of blocking the valves.

The tests showed that the quantity of product dispensed by the spray devices and the spray pattern of the spray applied remained substantially consistent over the 15 test days.

The results of the quantity dispensed and spray pattern tests confirmed that the compositions of the invention remained constant over time, allowing constant dispensing of the product and good cover of the affected area to be guaranteed.

## Claims

1. A composition comprising 15-25 wt% of sucralfate gel, 0.05-0.2 wt% of PVA, 0.5-2 wt% of sodium phosphate monobasic and at least 50 wt% of water, on the total composition weight.

2. The composition of claim 1, comprising 16-22 wt% of sucralfate gel, 0.08-0.15 wt% of PVA, 0.8-1.5 wt% of sodium phosphate monobasic and at least 50 wt% of water, on the total composition weight.

3. The composition of claim 2, comprising 17-20 wt% of sucralfate gel, 0.09-0.12 wt% of PVA, 0.9-1.2 wt% of sodium phosphate monobasic and at least 55 wt% of water, on the total composition weight.

4. The composition of claim 3, comprising 20 wt% of sucralfate gel, 0.1 wt% of PVA, 1 wt% of sodium phosphate monobasic and at least 75 wt% of water, on the total composition weight.

5. The composition of any one of claims 1-3, further comprising 0.75-8 wt% of tocotrienols, 0.1-2.5 wt% of tocopherols, and 1-5 wt% of a non-ionic surfactant.

6. The composition of claim 5, comprising 1.5-6 wt% of tocotrienols, 0.4-1.7 wt% of tocopherols, and 2-4 wt% of a non-ionic surfactant.

7. The composition of claim 6, comprising 1.5-4.5 wt% of tocotrienols, 0.45-1.4 wt% of tocopherols, and 2.5-3.5 wt% of a non-ionic surfactant.

8. The composition of claim 7, comprising 18 wt% of sucralfate gel, 1.975 wt% of tocotrienols, 0.575 wt% of tocopherols, 0.1 wt% of PVA, 1 wt% of sodium phosphate monobasic, 3 wt% of a non-ionic surfactant, and at least 72 wt% of water, on the total composition weight.

9. The composition of claim 7, comprising 17 wt% of sucralfate gel, 3.95 wt% of tocotrienols, 1.5 wt% of tocopherols, 0.1 wt% of PVA, 1 wt% of sodium phosphate monobasic, 3 wt% of a non-ionic surfactant, and at least 68 wt% of water, on the total composition weight.

10. The composition of claim 1 consisting of 15-25 wt% of sucralfate gel, 0.05-0.2 wt% of PVA, 0.5-2 wt% of sodium phosphate monobasic, 0-9 wt% of tocotrienols, 0-3 wt% of tocopherols, 0-6 wt% of a non-ionic surfactant, and at least 50 wt% of water, the remaining being pharmaceutically acceptable excipients, on the total composition weight.

11. The composition of any one of claims 1-10 for use in the treatment of cutaneous lesions.

12. The composition of claim 11, wherein said cutaneous lesions are maculae, papulae, vesicles, bullae, pustulae, cysts, erosions, abrasions, rash, ulcers, fissures, sores, telangectasia, scales, erythema, crusts, lichenifications, excoriations, indurations, cuts, lacerations, or atrophy.

13. The composition of claim 11 or 12, wherein said composition is topically sprayed on the lesion to be treated.

14. Process for the preparation of the composition of claim 1 comprising the steps of:
a) providing an aqueous suspension of sucralfate gel and sodium phosphate monobasic;
b) adding to said suspension PVA previously dissolved in water at a temperature of 70-100°C under stirring; and
c) homogenising the mixture resulting from step b) in a high-pressure homogenizer.

15. The process of claim 14, further comprising the steps of:
d) homogenising in a high-pressure homogenizer tocotrienols and tocopherols dispersed in a non-ionic surfactant;
e) adding the mixture of step d) to the aqueous suspension of step a); and
f) homogenising the mixture resulting from step e) in a high-pressure homogenizer.

## Patentansprüche

1. Zusammensetzung, umfassend 15-25 Gew.-% Sucralfatgel, 0,05-0,2 Gew.-% PVA, 0,5-2 Gew.-% Natriumdihydrogenphosphat und mindestens 50 Gew.-% Wasser, bezogen auf das Gesamtgewicht der Zusammensetzung.

2. Zusammensetzung gemäß Anspruch 1, umfassend 16-22 Gew.-% Sucralfatgel, 0,08-0,15 Gew.-% PVA, 0,8-1,5 Gew.-% Natriumdihydrogenphosphat und mindestens 50 Gew.-% Wasser, bezogen auf das Gesamtgewicht der Zusammensetzung.

3. Zusammensetzung gemäß Anspruch 2, umfassend 17-20 Gew.-% Sucralfatgel, 0,09-0,12 Gew.-% PVA, 0,9-1,2 Gew.-% Natriumdihydrogenphosphat und mindestens 55 Gew.-% Wasser, bezogen auf das Gesamtgewicht der Zusammensetzung.

4. Zusammensetzung gemäß Anspruch 3, umfassend 20 Gew.-% Sucralfatgel, 0,1 Gew.-% PVA, 1 Gew.-% Natriumdihydrogenphosphat und mindestens 75 Gew.-% Wasser, bezogen auf das Gesamtgewicht der Zusammensetzung.

5. Zusammensetzung gemäß einem der Ansprüche 1-3, des Weiteren umfassend 0,75-8 Gew.-% Tocotrienole, 0,1-2,5 Gew.-% Tocopherole und 1-5 Gew.-% eines nichtionischen Tensids.

6. Zusammensetzung gemäß Anspruch 5, umfassend 1,5-6 Gew.-% Tocotrienole, 0,4-1,7 Gew.-% Tocopherole und 2-4 Gew.-% eines nichtionischen Tensids.

7. Zusammensetzung gemäß Anspruch 6, umfassend 1,5-4,5 Gew.-% Tocotrienole, 0,45-1,4 Gew.-% Tocopherole und 2,5-3,5 Gew.-% eines nichtionischen Tensids.

8. Zusammensetzung gemäß Anspruch 7, umfassend 18 Gew.-% Sucralfatgel, 1,975 Gew.-% Tocotrienole, 0,575 Gew.-% Tocopherole, 0,1 Gew.-% PVA, 1 Gew.-% Natriumdihydrogenphosphat, 3 Gew.-% eines nichtionischen Tensids und mindestens 72 Gew.-% Wasser, bezogen auf das Gesamtgewicht der Zusammensetzung.

9. Zusammensetzung gemäß Anspruch 7, umfassend 17 Gew.-% Sucralfatgel 3,95 Gew.-% Tocotrienole 1,5 Gew.-% Tocopherole, 0,1 Gew.-% PVA, 1 Gew.-% Natriumdihydrogenphosphat, 3 Gew.-% eines nichtionischen Tensids und mindestens 68 Gew.-% Wasser, bezogen auf das Gesamtgewicht der Zusammensetzung.

10. Zusammensetzung gemäß Anspruch 1, umfassend 15-25 Gew.-% Sucralfatgel, 0,05-0,2 Gew.-% PVA, 0,5-2 Gew.-% Natriumdihydrogenphosphat, 0-9 Gew.-% Tocotrieneole, 0-3 Gew.-% Tocopherole, 0-6 Gew.-% eines nichtionischen Tensids und mindestens 50 Gew.-% Wasser, wobei der Rest des Gesamtgewichts der Zusammensetzung pharmazeutisch zulässige Hilfsstoffe sind.

11. Die Zusammensetzung gemäß einem der Ansprüche 1 bis 10 zur Verwendung bei der Behandlung von Hautläsionen.

12. Zusammensetzung gemäß Anspruch 11, wobei genannte Hautläsionen Flecken, Papula, Bläschen, Bullae, Pusteln, Zysten, Erosionen, Abrasionen, Ausschlag, Geschwüre, Fissuren, Wundstellen, Telangektasien, Schuppen, Erytheme, Schorf, Lichenifikationen, Abschürfungen, Gewebeverhärtungen, Schnittwunden, Lazerationen oder Atrophie sind.

13. Zusammensetzung gemäß Anspruch 11 oder 12, wobei genannte Zusammensetzung topisch auf die zu behandelnde Läsion gesprüht wird.

14. Verfahren zur Herstellung der Zusammensetzung gemäß Anspruch 1, umfassend die folgenden Schritte:
a) Bereitstellen einer wässrigen Suspension von Sucralfatgel und Natriumdihydrogenphosphat;
b) Zugeben von PVA, das vorher in Wasser gelöst wurde, zur genannten Suspension unter Rühren bei einer Temperatur von 70-100 °C; und
c) Homogenisieren der aus Schritt b) resultierenden Mischung in einem Hochdruck-Homogenisator.

15. Verfahren gemäß Anspruch 14, des Weiteren umfassend die folgenden Schritte:
d) Homogenisieren von Tocotrienolen und Tocopherolen, die in einem nichtionischen Tensid dispergiert sind, in einem Hochdruck-Homogenisator;
e) Zugeben der Mischung von Schritt d) zu der wässrigen Suspension von Schritt a); und
f) Homogenisieren der aus Schritt e) resultierenden Mischung in einem Hochdruck-Homogenisator.

## Revendications

1. Composition comprenant 15 à 25 % en poids de gel de sucralfate, 0,05 à 0,2 % en poids de PVA, 0,5 à 2 % en poids de phosphate de sodium monobasique et au moins 50 % en poids d'eau, par rapport au poids total de la composition.

2. Composition selon la revendication 1, comprenant 16 à 22 % en poids de gel de sucralfate, 0,08 à 0,15 % en poids de PVA, 0,8 à 1,5 % en poids de phosphate de sodium monobasique et au moins 50 % en poids d'eau, par rapport au poids total de la composition.

3. Composition selon la revendication 2, comprenant 17 à 20 % en poids de gel de sucralfate, 0,09 à 0,12 % en poids de PVA, 0,9 à 1,2 % en poids de phosphate de sodium monobasique et au moins 55 % en poids d'eau, par rapport au poids total de la composition.

4. Composition selon la revendication 3, comprenant 20 % en poids de gel de sucralfate, 0,1 % en poids de PVA, 1 % en poids de phosphate de sodium monobasique et au moins 75 % en poids d'eau, par rapport au poids total de la composition.

5. Composition selon l'une quelconque des revendications 1 à 3, comprenant en outre 0,75 à 8 % en poids de tocotriénols, 0,1 à 2,5 % en poids de tocophérols, et 1 à 5 % en poids d'un tensioactif non ionique.

6. Composition selon la revendication 5, comprenant 1,5 à 6 % en poids de tocotriénols, 0,4 à 1,7 % en poids de tocophérols, et 2 à 4 % en poids d'un tensioactif non ionique.

7. Composition selon la revendication 6, comprenant 1,5 à 4,5 % en poids de tocotriénols, 0,45 à 1,4 % en poids de tocophérols, et 2,5 à 3,5 % en poids d'un tensioactif non ionique.

8. Composition selon la revendication 7, comprenant 18 % en poids de gel de sucralfate, 1,975 % en poids de tocotriénols, 0,575 % en poids de tocophérols, 0,1 % en poids de PVA, 1 % en poids de phosphate de sodium monobasique, 3 % en poids d'un tensioactif non ionique, et au moins 72 % en poids d'eau, par rapport au poids total de la composition.

9. Composition selon la revendication 7, comprenant 17 % en poids de gel de sucralfate, 3,95 % en poids de tocotriénols, 1,5 % en poids de tocophérols, 0,1 % en poids de PVA, 1 % en poids de phosphate de sodium monobasique, 3 % en poids d'un tensioactif non ionique, et au moins 68 % en poids d'eau, par rapport au poids total de la composition.

10. Composition selon la revendication 1, se composant de 15 à 25 % en poids de gel de sucralfate, 0,05 à 0,2 % en poids de PVA, 0,5 à 2 % en poids de phosphate de sodium monobasique, 0 à 9 % en poids de tocotriénols, 0 à 3 % en poids de tocophérols, 0 à 6 % en poids d'un tensioactif non ionique, et au moins 50 % en poids d'eau, le reste étant constitué d'excipients pharmaceutiquement acceptables, par rapport au poids total de la composition.

11. Composition selon l'une quelconque des revendications 1 à 10 pour une utilisation dans le traitement des lésions cutanées.

12. Composition selon la revendication 11, lesdites lésions cutanées étant des macules, papules, vésicules, bulles, pustules, kystes, irritations, abrasions, éruption, ulcères, fissures, plaies, téléangectasies, squames, érythèmes, croûtes, lichénifications, excoriations, indurations, coupures, lacérations ou une atrophie.

13. Composition selon la revendication 11 ou 12, ladite composition étant étalée topiquement sur la lésion à traiter.

14. Procédé de préparation de la composition selon la revendication 1, comprenant les étapes consistant à :
a) fournir une suspension aqueuse de gel de sucralfate et de phosphate de sodium monobasique ;
b) ajouter à ladite suspension du PVA dissous au préalable dans de l'eau à une température de 70 à 100 °C sous agitation ; et
c) homogénéiser le mélange résultant de l'étape b) dans un homogénéiseur haute pression.

15. Procédé selon la revendication 14, comprenant en outre les étapes consistant à :
d) homogénéiser dans un homogénéiseur haute pression les tocotriénols et les tocophérols dispersés dans un tensioactif non ionique ;
e) ajouter le mélange de l'étape d) à la suspension aqueuse de l'étape a) ; et
f) homogénéiser le mélange résultant de l'étape e) dans un homogénéiseur haute pression.
